# EUROPÄISCHE PATENTSCHRIFT

(11) **EP 1 382 362 B1**
(45) Veröffentlichungstag und Bekanntmachung des Hinweises auf die Patenterteilung: **23.08.2006**
(21) Anmeldenummer: 03000665.4
(22) Anmeldetag: 16.01.2003
(51) Int. Cl.: A61M 5/145, B66D 1/39

(54) **Motorgetriebene Infusionspumpe**
Motor-driven infusion pump
Pompe à perfusion entraînée par un moteur

(30) Priorität: 17.07.2002 DE 10232408
(43) Veröffentlichungstag der Anmeldung: 21.01.2004
(73) Patentinhaber: Pegasus GmbH, Gesellschaft für medizinische Geräte, 24118 Kiel (DE)
(72) Erfinder: Kölln, Harm, 24159 Kiel (DE)
(74) Vertreter: UEXKÜLL & STOLBERG

(56) Entgegenhaltungen:
- US-A- 2 592 381
- US-A- 2 833 280
- US-A- 3 804 370
- PATENT ABSTRACTS OF JAPAN vol. 1998, no. 02, 30. Januar 1998 (1998-01-30) & JP 09 262293 A (TERUMO CORP), 7. Oktober 1997 (1997-10-07)

## Beschreibung

Die Erfindung bezieht sich auf eine motorgetriebene Infusionspumpe mit einer in sie eingesetzten Kolbenspritze, die im Innenraum ihres Gehäuses einen in Richtung auf ihre Abgabeöffnung bewegbaren Kolben aufweist, der mittels der vom Motor erzeugten Antriebskraft gesteuert verlagerbar ist, sowie auf eine Kolbenspritze zum Einsatz in einer Infusionspumpe.

Derartige Infusions- oder Spritzenpumpen werden in großem Umfang in Krankenhäusern, aber auch in Notarztwagen, Rettungshubschraubern u.ä. eingesetzt, um einem Patienten über längere Zeit gesteuert ein Medikament zu injizieren. Hierzu wird eine mit dem Medikament gefüllte Kolbenspritze in die Infusionsspritze eingelegt und festgeklemmt und ihre Abgabeöffnung über einen Schlauch und eine Infusionsnadel mit dem Patienten verbunden. Die aus dem hinteren Ende des Gehäuses der Kolbenspritze vorstehende Kolbenstange wird dann zur gesteuerten Abgabe des Medikaments von einem entsprechend gesteuerten Kolben der Infusionspumpe in diese hineinverlagert, so dass der Kolben der Kolbenspritze das Medikament aus dieser herausdrückt.

Diese bekannten Infusionspumpen arbeiten zufriedenstellend, haben jedoch den Nachteil, dass sie verhältnismäßig große Abmessungen haben, da sie die gesamte Kolbenspritze aufnehmen müssen, die im gefüllten Zustand infolge der nach hinten herausstehenden Kolbenstange mehr als die doppelte Länge des Raums hat, der mit dem Medikament gefüllt ist.

Zur Reduzierung der Abmessungen der Infusionspumpe ist es auch bekannt bekannt (JP 9-262293 A), die Kolbenspritze so auszubilden, dass deren Kolben nicht durch Beaufschlagung einer nach hinten aus dem Gehäuse der Kolbenspritze hervorstehenden Kolbenstange verlagert wird, sondern durch gesteuertes Aufwickeln eines Zugelements, das mittig am Kolben befestigt ist und diesen in Richtung auf die Vorderwand des Gehäuses der Kolbenspritze und damit auf deren Abgabeöffnung bewegt. Hierzu wird das Zugelement auf die glatte zylindrische Oberfläche eines vom Motor der Infusionspumpe drehend angetriebenen Aufnahmeschafts aufgewickelt, wobei es jedoch während des Aufwickelvorgangs zur Überlagerung von Windungen des Zugelements kommen kann. Dann vergrößert sich für den noch aufzuwickelnden Abschnitt des Zugelements der effektive Durchmesser des Aufnahmeschafts und damit die Vorschubgeschwindigkeit des Kolbens, was zu einer Veränderung der pro Zeiteinheit ausgetragenen Menge an Medikament führt. Diese bekannte Kolbenspritze hat daher zwar eine deutlich geringere Abmessung als die bisher üblicherweise eingesetzten Kolbenspritzen mit Kolbenstange, so dass auch die Infusionspumpe eine deutliche reduzierte Abmessung haben kann, doch ergeben sich im Betrieb Schwankungen der Menge an ausgetragenem Medikament.

Es ist Aufgabe der Erfindung, eine derartige Infusionspumpe so auszubilden, dass der Kolben der Kolbenspritze im Betrieb mit weitestgehend konstanter Vorschubgeschwindigkeit verlagert wird.

Zur Lösung dieser Aufgabe dient eine Infusionspumpe gemäß Anspruch 1. Eine Kolbenspritze zum Einsatz in einer Solchen Infusionspumpe ist in Anspruch 8 beansprucht.

Bei der erfindungsgemäßen Infusionspumpe weist somit der Aufnahmeschaft einen Gewindebereich auf, der in Eingriff mit dem Innengewinde einer am Gehäuse vorgesehenen Spindelführung steht, und der Aufnahmeschaft ist formschlüssig und axial verlagerbar mit der Antriebswelle verbunden. Auf diese Weise ergibt eine Drehung des Aufnahmeschafts durch die Antriebswelle der Infusionspumpe eine axiale Verlagerung des Aufnahmeschafts, d. h. der Aufwickelbereich des Aufnahmeschafts verlagert sich relativ zur Mittelachse des Gehäuses, wodurch für die Aufnahme des Zugelements immer ein noch nicht mit dem Zugelement belegter Bereich des Aufwickelbereichs zur Verfügung steht, die Ausrichtung des Zugelements bezüglich der Austrittsöffnung also nicht durch Überlagerungen von mehreren Windungen des Zugelements auf dem Aufnahmeschaft verändert wird. Auf diese Weise wird erreicht, dass der Kolben mit konstanter Vorschubgeschwindigkeit verfahren und immer die gleiche Menge an Medikament pro Zeiteinheit ausgetragen wird.

Um das Zugelement möglichst reibungsarm zu verlagern, kann dieses tangential an den Aufnahmeschaft herangeführt sein, d.h. die Mittelachse des Gehäuses bildet eine Tangente an den Aufwickelbereich des Aufnahmeschafts.

Vorzugsweise erstreckt sich der Gewindebereich des Aufnahmeschafts bis in den Aufwickelbereich für das Zugelement, so dass sich das Zugelement bei Drehung des Aufnahmeschafts in den Grund des auf ihm vorgesehenen Gewindegangs legt.

Ein besonders einfacher Aufbau der Kolbenspritze ergibt sich, wenn die Spindelführung von der die Austrittsöffnung aufweisenden Vorderwand des Gehäuses abstehend einstückig mit dieser ausgebildet ist. Das Gehäuse einer derartigen Kolbenspritze kann auf einfache Weise einstückig aus Kunststoff hergestellt werden.

Um den Motor der Infusionspumpe so einbauen zu können, dass das Gehäuse der Infusionspumpe möglichst klein bleibt, d.h. um eine Anordnung des Motors in einer Ebene parallel zur eingesetzten Kolbenspritze zu ermöglichen, kann die Abgabewelle des Motors mit der Antriebswelle zur Drehung des Aufnahmeschafts der Kolbenspritze über ein Schneckengetriebe gekoppelt sein.

Da die Austrittsöffnung für das Zugelement mittig in der Vorderwand des Gehäuses der Kolbenspritze angeordnet ist, wird die Abgabeöffnung für den Spritzeninhalt vorzugsweise außermittig in der Vorderwand des Gehäuses vorgesehen.

Der Kolben der Kolbenspritze besteht vorzugsweise aus einem dichtend an der Gehäusewand anliegenden vorderen Abschnitt aus Material mit kautschukartigen Eigenschaften und einem mit diesem fest verbundenen, formstabilen hinteren Abschnitt, so dass die Form und das Material des vorderen Abschnitts allein danach ausgewählt werden kann, dass es einerseits resistent gegen das mit ihm in Berührung kommende Medikament ist und andererseits die optimalen Materialeigenschaften hat, um eine wirksame Abdichtung mit einer guten Verlagerbarkeit im Gehäuse zu kombinieren, während die Formstabilität allein durch den hinteren Abschnitt sichergestellt wird.

Wenn bei einem solchen Aufbau die Kolbenspritze vom Arzt mit dem gewünschten Medikament befüllt werden soll, kann der hintere Abschnitt des Kolbens formschlüssig, jedoch lösbar mit einer das Zurückziehen des Kolbens ermöglichenden Kolbenstange verbindbar sein, die es dem Arzt ermöglicht, in für konventionelle Kolbenspritzen üblicher Weise, das gewünschte Medikament aus einer Ampulle o.ä. durch Zurückziehen des sich in seiner vorderen Stellung befindenden Kolbens in das Gehäuse der Kolbenspritze zu saugen. Danach wird dann die Kolbenstange entfernt und die Kolbenspritze in der vorstehend erläuterten Weise in die Infusionspumpe eingesetzt.

Wenn die Kolbenspritze dazu vorgesehen ist, vom Arzt mit einem gewünschten Medikament befüllt zu werden, so dass sich der Kolben in der unbefüllten Ausgangsstellung benachbart zur Vorderwand des Gehäuses befindet, ist es vorteilhaft, wenn sich der bei sich vollständig zurückgezogenem Kolben im Inneren des Gehäuses erstreckende Teil des Zugelements in dieser unbefüllten Ausgangsstellung zwischen Kolben und Vorderwand des Gehäuses befindet. Auf diese Weise ist sichergestellt, dass der gesamte, später mit dem Medikament in Berührung kommende Teil des Zugelements sich dauernd in dem sterilen Raum innerhalb des Gehäuses der Kolbenspritze befindet, also nicht die Gefahr einer Verunreinigung des aufzusaugenden Medikaments besteht.

Die Erfindung wird im Folgenden anhand der ein Ausführungsbeispiel zeigenden Figuren näher erläutert.
- Figur 1: zeigt in einer auseinandergezogenen schematischen Darstellung die Infusionspumpe und die zugehörige Kolbenspritze mit Kolbenstange.
- Figur 2: zeigt die Kolbenspritze aus Figur 1 in der Ausgangsstellung des Kolbens mit eingesetzter Kolbenstange.
- Figur 3: zeigt die Kolbenspritze aus Figur 1 in einer perspektivischen, teilweise aufgebrochenen Darstellung.
- Figur 4: zeigt vergrößert den in Figur 3 eingekreisten Bereich der Kolbenspritze.
- Figur 5: zeigt eine Ansicht der Infusionspumpe mit eingesetzter, gefüllter Kolbenspritze.
- Figur 6: zeigt einen Schnitt entlang der Linie VI-VI aus Figur 5.
- Figur 7: zeigt in einer Darstellung gemäß Figur 6 die Kolbenspritze im vollständig entleerten Zustand.

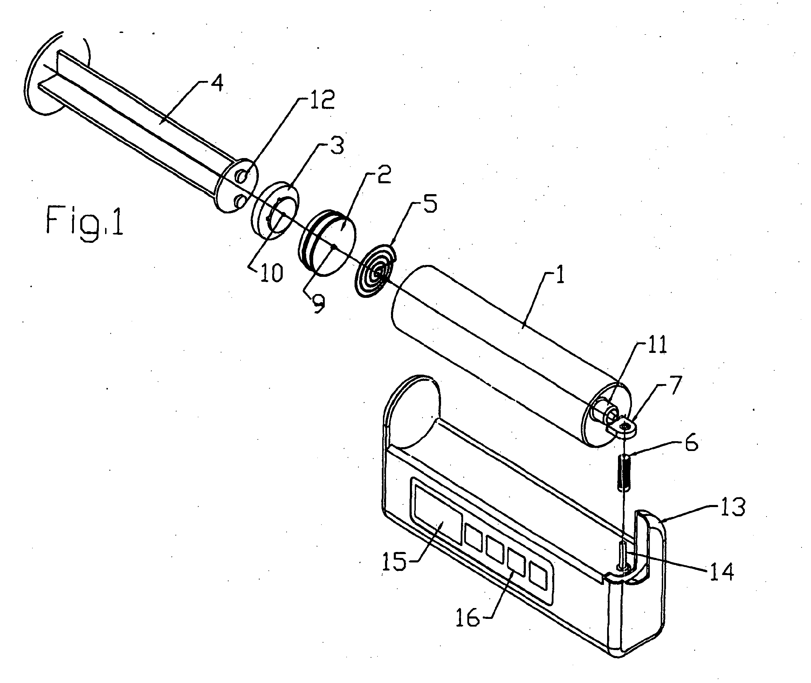

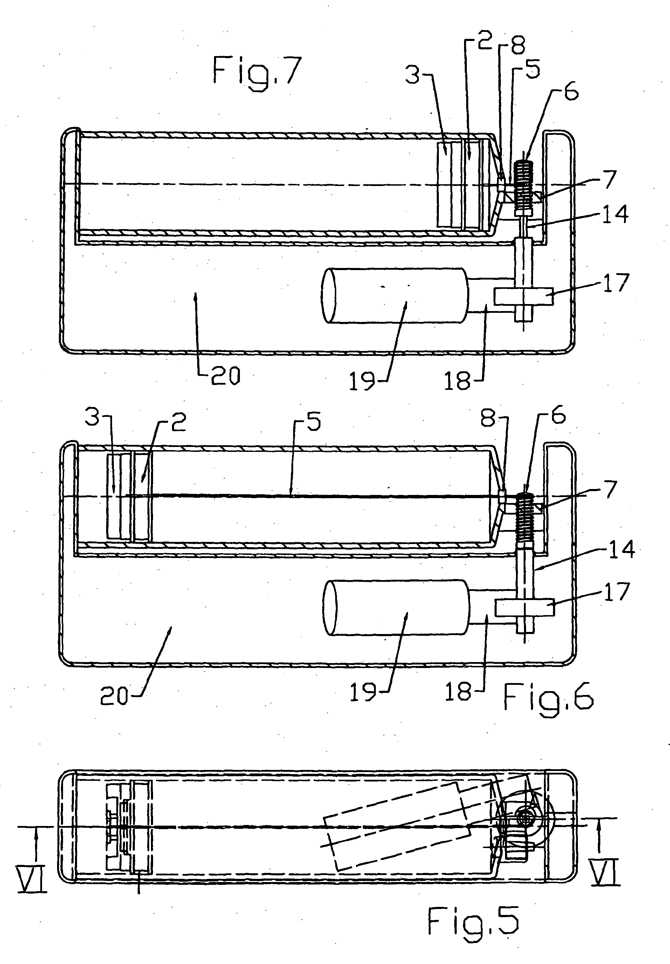

In Figur 1 ist schematisch eine Infusionspumpe mit einem vorzugsweise aus Kunststoff bestehenden Gehäuse 13 gezeigt, die einen Antriebsmotor 19 (Figuren 5 bis 7) sowie die für diesen Antriebsmotor bei derartigen Infusionspumpen übliche, nicht dargestellte Steuerung ggf. mit Schnittstelle für eine Datenübertragung einschließlich schematisch angedeutetem Display 15 und Tastenfeld 16 aufweist. Im oberen Bereich bildet das Gehäuse 13 der Infusionspumpe eine Aufnahme für eine noch zu beschreibende Kolbenspritze.

Der Motor 19 ist mit seiner Längsachse in einer Ebene parallel zur Längsachse der in das Gehäuse 13 eingesetzten Kolbenspritze liegend angeordnet und trägt auf seiner Ankerwelle eine Schnecke 18, die mit einem Schneckenrad 17 kämmt. Das Schneckenrad 17 ist auf einer Antriebswelle 14 befestigt, die bezogen auf die Standfläche des Gehäuses 13 der Pumpe senkrecht nach oben durch die Auflagefläche für die Kolbenspritze vorsteht.

Die Kolbenspritze hat ein übliches, zylindrisches Gehäuse 1, das vorzugsweise aus Kunststoff besteht und das am hinteren Ende offen ist, während sich in seiner Vorderwand außermittig eine Abgabeöffnung für den Spritzeninhalt, hier in Form eines sogenannten Luer Lock 11, befindet. Mittig ist in der Vorderwand eine Austrittsöffnung 8 vorgesehen, durch die gegen den Durchtritt von Spritzeninhalt abgedichtet der noch zu beschreibende Faden 5 geführt ist. Ferner ist an der Vorderwand unterhalb der Abgabeöffnung 8 einstückig mit dem Gehäuse eine Spindelführung 7 ausgebildet, die eine Gewindebohrung aufweist, in die ein ein Außengewinde aufweisender Aufnahmeschaft 6 eingeschraubt ist. Dieser hat an seinem unteren Ende eine Aufnahmeöffnung für das obere Ende der Antriebswelle 14, durch die sich ein formschlüssiger Eingriff zwischen Antriebswelle 14 und Aufnahmeschaft 6 ergibt, die jedoch eine axiale Verlagerung des Aufnahmeschafts 6 bezüglich der Antriebswelle 14 gestattet.

Der Kolben der Kolbenspritze besteht aus einem vorderen Abschnitt 2, der aus dem für Kolben von derartigen Kolbenspritzen üblichen Kautschuk oder kautschukartigen Material besteht, das eine dichtende, jedoch verschiebbare Anlage an der Innenfläche des Gehäuses 1 ermöglicht, sowie aus einen fest mit dem vorderen Abschnitt 2 verbundenen hinteren Abschnitt 3 aus formstabilem Kunststoff. An einer in Form eines Vorsprungs ausgebildeten Halterung 10 des hinteren Abschnitts 3 des Kolbens, auf die der vordere Abschnitt 2 aufgesetzt ist, ist in nicht dargestellter Weise ein Seil 5 befestigt, das durch eine Mittelöffnung 9 im vorderen Abschnitt 2 des Kolbens geführt ist und mit einem vorderen Bereich durch die Abgabeöffnung 8 hindurchtritt. Der außerhalb dieser Abgabeöffnung liegende Abschnitt des Fadens 5 ist mit dem Aufnahmeschaft 6 verbunden, dessen Gewinde sich über praktisch seine gesamte Länge erstreckt, so dass der Faden 5 an einem mit Gewinde versehenen Bereich des Aufnahmeschafts 6 angreift. Dabei ist die Gewindebohrung in der Spindelführung 7 und damit die Lage des Aufnahmeschafts 6 so gewählt, dass die Längsmittelachse des Gehäuses 1 der Kolbenspritze im in die Infusionspumpe eingesetzten Zustand tangential am äußeren Umfang des Aufnahmeschafts 6 liegt, wie dies in Figur 5 zu erkennen ist. Der Faden 5 legt sich also entsprechend tangential an den Aufnahmeschaft 6 an.

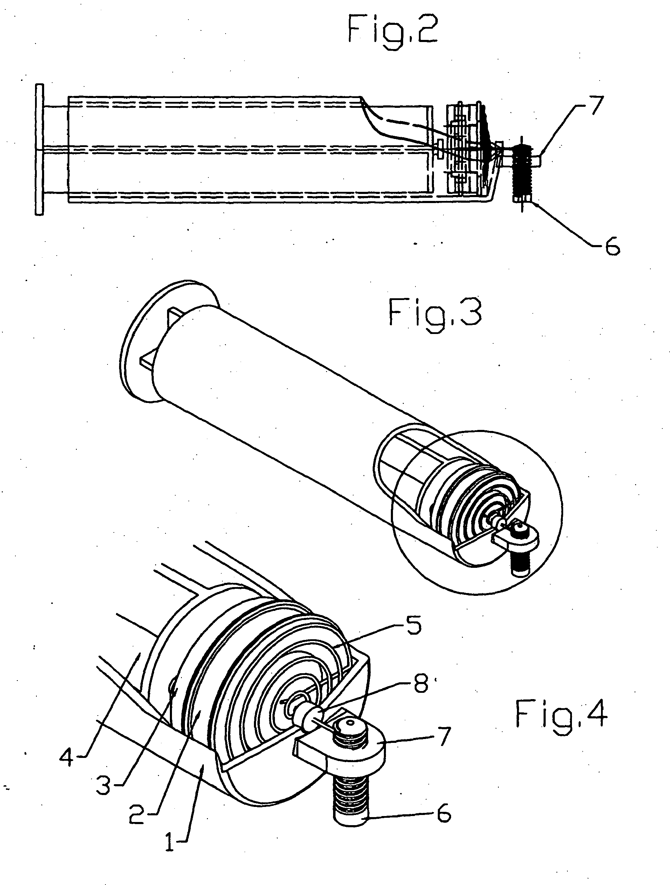

Wird die Kolbenspritze vom Hersteller unbefüllt geliefert, um vom Arzt mit dem gewünschten Medikament befüllt zu werden, so befindet sich der Kolben 2, 3, wie in den Figuren 2 bis 4 gezeigt, unmittelbar benachbart zur Vorderwand des Gehäuses 1, und der gesamte zu diesem Zeitpunkt nicht benötigte Abschnitt des Fadens 5 liegt im Inneren des Gehäuses 1, also im sterilen Raum zwischen Vorderwand des Gehäuses 1 und dem vorderen Abschnitt 2 des Kolbens 2, 3.

Um die Kolbenspritze befüllen zu können, sind in der Rückseite des hinteren Abschnitts 3 des Kolbens 2, 3 bayonettartige Öffnungen vorgesehen, in die entsprechende Vorsprünge 12 einer Kolbenstange 4 eingreifen können. Zum Befüllen wird die Kolbenstange 4 von hinten in das Gehäuse 1 eingeschoben und mit dem Kolben verbunden, worauf in üblicher Weise eine auf das Luer Lock 11 aufgesetzte Kanüle in das aufzusaugende Medikament getaucht und der Kolben 2, 3 durch Zurückziehen der Kolbenstange zurückgezogen und so das Medikament in das Gehäuse 1 gesaugt wird.

Die auf diese Weise befüllte Kolbenspritze wird in die Infusionspumpe eingesetzt, und durch geringfügiges Drehen der Antriebswelle 14 und damit des Aufnahmeschafts 6 wird Faden 5 auf den Aufnahmeschaft aufgewickelt, bis sein sich zwischen Aufnahmeschaft 6 und Kolben 2, 3 erstreckender Abschnitt gespannt ist. Die Kolbenspritze wird dann in üblicher Weise über einen Schlauch und eine Kanüle mit dem Patienten verbunden. Wenn der Motor 19 in bekannter Weise gesteuert aktiviert wird, ergibt sich durch das Aufwickeln des Fadens 5 auf den Aufnahmeschaft 6 eine entsprechend gesteuerte Verlagerungsbewegung des Kolbens 2, 3 und damit die gewünschte gesteuerte Abgabe von Medikament aus der Kolbenspritze. Da dabei der Aufnahmeschaft 6 infolge seiner Gewindekopplung mit dem Innengewinde der Durchgangsbohrung der Spindelführung 7 kontinuierlich nach oben verlagert wird (vgl. Stellungen gemäß Figuren 6 und 7), wickelt sich der Faden 5 entsprechend durch Einlagerung in den Gewindegang auf der Außenfläche des Aufnahmeschafts 6 auf diesen auf, d.h. der Faden 5 legt sich während des gesamten Aufwickelvorgangs immer tangential an den äußeren Umfang des Aufnahmeschafts 6 an, so dass sein zwischen Aufnahmeschaft 6 und Kolben 2, 3 liegender Bereich immer genau auf der Mittelachse der Kolbenspritze angeordnet bleibt. Daher ergeben sich keinerlei Ungenauigkeiten bei der Vorschubbewegung des Kolbens 2, 3, wie sie sonst durch Umlenkungen des Fadens 5 eintreten könnten.

Es sei darauf hingewiesen, dass es selbstverständlich auch möglich ist, die Kolbenspritze mit Medikament befüllt zu liefern, so dass sich dann die Ausgangsstellung des Kolbens 2, 3 gemäß Figuren 5 und 6 ergibt und weder eine Kolbenstange noch eine entsprechende Eingriffsausbildung für die Kolbenstange an der Rückseite des hinteren Abschnitts 3 des Kolbens 2, 3 benötigt wird.

## Patentansprüche

1. Motorgetriebene Infusionspumpe mit einer in sie eingesetzten Kolbenspritze, die im Innenraum ihres Gehäuses (1) einen in Richtung auf ihre Abgabeöffnung (11) bewegbaren Kolben (2, 3) aufweist, der mittels der vom Motor (19) erzeugten Antriebskraft mit Hilfe eines Zugelements (5) gesteuert verlagerbar ist, das am Abgabeende des Gehäuse (1) der Kolbenspritze durch eine koaxial zur Längsmittelachse des Gehäuses (1) angeordnete Austrittsöffnung (8) abgedichtet herausgeführt ist und auf einen mittels einer am Gehäuse (1) vorgesehenen Spindelführung (7) drehbar und quer zur Längsmittelachse des Gehäuses (1) an diesem gehalterten Aufnahmeschaft (6) aufwickelbar ist, der in lösbarem, formschlüssigem Antriebseingriff mit einer vom Motor (19) angetriebenen Antriebswelle (14) der Infusionspumpe steht,
**dadurch gekennzeichnet,**
**dass** der Aufnahmeschaft (6) einen Gewindebereich aufweist, der in Eingriff mit einem Innengewinde der Spindelführung (7) steht, und dass der Aufnahmeschaft (6) axial verlagerbar mit der Antriebswelle (14) verbunden ist, so dass das Zugelement ohne Veränderung seiner Ausrichtung zum Aufnahmeschaft (6) auf diesen aufgewickel bar ist.

2. Infusionspumpe nach Anspruch 1, **dadurch gekennzeichnet, dass** das Zugelement (5) tangential an den Aufnahmeschaft (6) herangeführt ist.

3. Infusionspumpe nach Anspruch 1 oder 2, **dadurch gekennzeichnet, dass** sich der Gewindebereich des Aufnahmeschafts (6) bis in den Aufwickelbereich für das Zugelement (5) erstreckt.

4. Infusionspumpe nach einem der Ansprüche 1 bis 3, **dadurch gekennzeichnet, dass** die Spindelführung (7) von der die Austrittsöffnung (11) aufweisenden Vorderwand des Gehäuses (1) abstehend einstückig mit diesem ausgebildet ist.

5. Infusionspumpe nach einem der Ansprüche 1 bis 4, **dadurch gekennzeichnet, dass** die Abgabewelle des Motors (19) über ein Schneckengetriebe (17, 18) mit der Antriebswelle (14) gekoppelt ist.

6. Infusionspumpe nach einem der Ansprüche 1 bis 5, **dadurch gekennzeichnet, dass** die Abgabeöffnung (11) für den Spritzeninhalt außermittig in der Vorderwand des Gehäuses (1) vorgesehen ist.

7. Infusionspumpe nach einem der Ansprüche 1 bis 6, **dadurch gekennzeichnet, dass** das Zugelement ein Faden (5) ist.

8. Kolbenspritze zum Einsatz in einer Infusionspumpe gemäß einem der Ansprüche 1 bis 7, mit
- einem länglichen Gehäuse (1), das am hinteren Ende offen ist und in seiner vorderen Endwand eine koaxial zur Mittelachse des Gehäuses (1) angeordnete Durchtrittsöffnung (8) aufweist,
- einem im Gehäuse (1) angeordneten Kolben (2, 3), der zum Herausdrücken des Spritzeninhalts durch eine im Gehäuse (1) vorgesehene Abgabeöffnung (11) in Richtung auf die vordere Endwand verlagerbar ist,
- einer am Gehäuse (1) vorgesehenen Spindelführung (7)
- einem in des Spindelführung geführten Aufnahmeschaft (6), der einen Aufwickelbereich für ein Zugelement (5) aufweist und mit einem Kopplungsbereich zur drehfesten Kopplung mit der Antriebswelle (14) der Infusionspumpe versehen ist, und
- einem Zugelement (5), das mittig an der Vorderseite des Kolbens (2, 3) befestigt, gegen Austritt von Spritzeninhalt durch die Durchtrittsöffnung (8) abgedichtet durch diese geführt und mit dem Aufwickelbereich des Aufnahmeschafts (6) verbunden ist, **dadurch gekennzeichnet, dass** die Spindelführung (7) eine mit Innengewinde versehene, mit ihrer Mittelachse senkrecht zur Längsachse des Gehäuses (1) angeordnete Durchgangsbohrung aufweist, der Aufnahmeschaft (6) mit einem an ihm vorgesehenen Gewindebereich in verdrehbarem Eingriff mit dem Innengewinde der Spindelführung (7) steht, und der Kopplungsbereich des Aufnahmeschafts (6) so ausgebildet ist, dass eine axiale Verlagerung zur Antriebswelle der Infusionspumpe möglich ist.

9. Kolbenspritze nach Anspruch 8, **dadurch gekennzeichnet, dass** die Mittelachse des Gehäuses (1) eine Tangente an den Aufwickelbereich des Aufnahmeschafts (6) bildet.

10. Kolbenspritze nach Anspruch 8 Oder 9, **dadurch gekennzeichnet, dass** sich der Gewindebereich des Aufnahmeschafts (6) bis in den Aufwickelbereich erstreckt.

11. Kolbenspritze nach einem der Ansprüche 8 bis 10, **dadurch gekennzeichnet, dass** die Spindelführung (7) von der die Austrittsöffnung (11) aufweisenden vorderen Endwand des Gehäuses (1) abstehend einstückig mit diesem ausgebildet ist.

12. Kolbenspritze nach einem der Ansprüche 8 bis 11, **dadurch gekennzeichnet, dass** die Abgabeöffnung (11) für den Spritzeninhalt außermittig in der vorderen Endwand des Gehäuses (1) vorgesehen ist.

13. Kolbenspritze nach einem der Ansprüche 8 bis 12, **dadurch gekennzeichnet, dass** das Zugelement ein Faden (5) ist.

14. Kolbenspritze nach einem der Ansprüche 8 bis 13, **dadurch gekennzeichnet, dass** der Kolben (2, 3) aus einem dichtend an der Gehäusewand anliegenden vorderen Abschnitt (2) aus Material mit kautschukartigen Eigenschaften und einem mit diesem fest verbundenen, formstabilen hinteren Abschnitt (3) besteht.

15. Kolbenspritze nach Anspruch 14, **dadurch gekennzeichnet, dass** der hintere Abschnitt (3) des Kolbens (2, 3) formschlüssig mit einer das Zurückziehen des Kolbens ermöglichenden Kolbenstange (4) verbindbar ist.

16. Kolbenspritze nach Anspruch 15, **dadurch gekennzeichnet, dass** sich der Kolben in der unbefüllten Ausgangsstellung benachbart zur vorderen Endwand des Gehäuses (1) befindet und dass sich der sich bei vollständig zurückgezogenem Kolben im Inneren des Gehäuses (1) erstreckende Teil des Zugelements (5) in der unbefüllten Ausgangsstellung zwischen Kolben und Vorderwand des Gehäuses (1) befindet.

## Claims

1. Motor-driven infusion pump comprising a syringe inserted therein, which, in the interior of its housing (1), has a piston (2, 3) which can be moved in the direction of its delivery opening (11) and which can be displaced in a controlled manner by means of the drive force generated by the motor (19) with the aid of a traction element (5), which is guided out in a sealed manner at the delivery end of the housing (1) of the syringe through an outlet opening (8) arranged coaxially to the longitudinal centre line of the housing (1) and can be wound onto a receiving shaft (6) which is held so as to be rotatable by means of a spindle guide (7) provided on the housing (1) and held thereon transversely to the longitudinal centre line of the housing (1), the receiving shaft being in releasable, positive drive engagement with a drive shaft (14), driven by the motor (19), of the infusion pump, **characterised in that** the receiving shaft (6) has a threaded region, which is in engagement with an internal thread of the spindle guide (7) and **in that** the receiving shaft (6) is connected, so as to be axially displaceable, to the drive shaft (14), so, without changing its orientation with respect to the receiving shaft (6), the traction element can be wound thereon.

2. Infusion pump according to claim 1, **characterised in that** the traction element (5) is guided tangentially onto the receiving shaft (6).

3. Infusion pump according to either claim 1 or claim 2, **characterised in that** the threaded region of the receiving shaft (6) extends into the winding region for the traction element (5).

4. Infusion pump according to any one of claims 1 to 3, **characterised in that** the spindle guide (7) is configured projecting from the leading wall of the housing (1) having the outlet opening (11) and in one piece therewith.

5. Infusion pump according to any one of claims 1 to 4, **characterised in that** the delivery shaft of the motor (19) is coupled to the drive shaft (14) via a worm gear (17, 18).

6. Infusion pump according to any one of claims 1 to 5, **characterised in that** the delivery opening (11) for the syringe contents is provided eccentrically in the leading wall of the housing (1).

7. Infusion pump according to any one of claims 1 to 6, **characterised in that** the traction element is a thread (5).

8. Syringe for use in an infusion pump according to any one of claims 1 to 7, comprising
- an elongate housing (1), which is open at the trailing end and has, in its leading end wall, a through-opening (8) arranged coaxially to the centre line of the housing (1),
- a piston (2, 3) which is arranged in the housing (1) and can be displaced in the direction of the leading end wall to press out the syringe contents through a delivery opening (11) provided in the housing (1),
- a spindle guide (7) provided on the housing (1),
- a receiving shaft (6) which is guided in the spindle guide and has a winding region for a traction element (5), and is provided with a coupling region for coupling in a rotationally engaged manner to the drive shaft (14) of the infusion pump, and
- a traction element (5), which, fastened centrally on the leading side of the piston (2, 3), is guided through the through-opening so as to be sealed against the escape of syringe contents through the through-opening (8) and is connected to the winding region of the receiving shaft (6),
**characterised in that** the spindle guide (7) has a through-hole provided with an internal thread and arranged with its centre line perpendicular to the longitudinal axis of the housing (1), the receiving shaft (6), with a threaded region provided thereon, is in rotatable engagement with the internal thread of the spindle guide (7) and the coupling region of the receiving shaft (6) is configured such that an axial displacement is possible with respect to the drive shaft of the infusion pump.

9. Syringe according to claim 8, **characterised in that** the centre line of the housing (1) forms a tangent to the winding region of the receiving shaft (6).

10. Syringe according to claim either 8 or claim 9, **characterised in that** the threaded region of the receiving shaft (6) extends into the winding region.

11. Syringe according to any one of claims 8 to 10, **characterised in that** the spindle guide (7) is configured so as to project from the leading end wall of the housing (1) having the outlet opening (11) and is configured in one piece therewith.

12. Syringe according to any one of claims 8 to 11, **characterised in that** the delivery opening (11) for the syringe contents is provided eccentrically in the leading end wall of the housing (1).

13. Syringe according to any one of claims 8 to 12, **characterised in that** the traction element is a thread (5).

14. Syringe according to any one of claims 8 to 13, **characterised in that** the piston (2, 3) consists of a leading portion (2) resting in a sealing manner on the housing wall and made of material with rubber-like properties and a dimensionally stable rear portion (3) rigidly connected thereto.

15. Syringe according to claim 14, **characterised in that** the rear portion (3) of the piston (2, 3) can be positively connected to a piston rod (4) allowing the piston to be pulled back.

16. Syringe according to claim 15, **characterised in that** the piston, in the unfilled starting position, is located adjacent to the leading end wall of the housing (1) and **in that** the part of the traction element (5) extending in the interior of the housing (1) when the piston is completely pulled back, is located, in the unfilled starting position, between the piston and leading wall of the housing (1).

## Revendications

1. Pompe de perfusion entraînée par un moteur, comprenant une seringue à piston installée dans cette pompe, seringue qui comporte, dans l'espace intérieur de son corps (1), un piston (2, 3) qui peut être déplacé en direction de son orifice de distribution (11), et qui peut être déplacé de façon contrôlée à l'aide d'une force motrice générée par un moteur (19), par l'intermédiaire d'un élément de traction (5) qui ressort de façon étanche par l'extrémité de distribution du corps (1) de la seringue à piston, à travers une ouverture de sortie (8) disposée coaxialement à l'axe longitudinal central du corps (1), et qui peut être enroulé sur un tambour récepteur (6) monté de façon rotative sur le corps (1) par l'intermédiaire d'un guidage de broche (7) prévu sur le corps (1), et perpendiculairement à l'axe longitudinal central de celui-ci, ce tambour étant en prise d'entraînement débrayable, par conjugaison des formes, avec un arbre d'entraînement (14) de la pompe de perfusion, qui est mû par le moteur (19),
**caractérisée en ce que** le tambour récepteur (6) comporte une partie filetée qui est en prise avec un filetage intérieur du guidage de broche (7), et que le tambour récepteur (6) est relié à l'arbre d'entraînement (14) de façon axialement mobile, de façon telle que l'élément de traction puisse être enroulé sur le tambour récepteur (6) sans modification de son alignement par rapport à celui-ci.

2. Pompe de perfusion selon la revendication 1, **caractérisée en ce que** l'élément de traction (5) est amené tangentiellement au tambour récepteur (6).

3. Pompe de perfusion selon la revendication 1 ou 2, **caractérisée en ce que** la zone filetée du tambour récepteur (6) s'étend jusque dans la partie d'enroulement de l'élément de traction (5).

4. Pompe de perfusion selon l'une des revendications 1 à 3, **caractérisée en ce que** le guidage de broche (7) est conformé d'un seul tenant avec, en faisant saillie depuis, la paroi frontale du corps (1) portant l'orifice de distribution (11).

5. Pompe de perfusion selon l'une des revendications 1 à 4, **caractérisée en ce que** l'arbre de sortie du moteur (19) est couplé à l'arbre d'entraînement (14) par l'intermédiaire d'un entraînement à vis sans fin (17, 18).

6. Pompe de perfusion selon l'une des revendications 1 à 5, **caractérisée en ce que** l'orifice de distribution (11) du contenu de la seringue est prévu de façon excentrée dans la paroi frontale du corps (1).

7. Pompe de perfusion selon l'une des revendications 1 à 6, **caractérisée en ce que** l'élément de traction est un fil (5).

8. Seringue à piston destinée à être utilisée dans une pompe de perfusion selon l'une des revendications 1 à 7, comprenant
- un corps allongé (1) dont l'extrémité arrière est ouverte et dont la paroi d'extrémité frontale comporte une ouverture débouchante (8) disposée coaxialement à l'axe central du corps (1),
- un piston (2, 3) disposé dans le corps (1) et pouvant être déplacé en direction de la paroi d'extrémité frontale lors de l'expulsion du contenu de la seringue à travers un orifice de distribution (11) ménagé dans le corps (1),
- un guidage de broche (7) prévu sur le corps (1),
- un tambour récepteur (6) guidé dans le guidage de broche, qui comporte une zone d'enroulement pour un élément de traction (5) et est pourvu d'une zone d'accouplement permettant l'accouplement, solidaire en rotation, à l'arbre d'entraînement (14) de la pompe de perfusion, et
- un élément de traction (5) qui est fixé de façon centrée sur la face avant du piston (2, 3), et qui est enfilé à travers l'ouverture de sortie (8) de façon étanche pour empêcher toute fuite du contenu de la seringue à travers cette ouverture, et est relié à la zone d'enroulement du tambour récepteur (6),
**caractérisée en ce que** le guidage de broche (7) comporte un trou débouchant pourvu d'un filetage intérieur dont l'axe central est disposé perpendiculairement à l'axe longitudinal du corps (1), que le tambour récepteur (6) est en prise, de façon à pouvoir tourner, par l'intermédiaire d'une zone filetée ménagée sur le tambour, avec le filetage intérieur du guidage de broche (7), et que la zone d'accouplement du tambour récepteur (6) est configurée de manière telle qu'un déplacement axial par rapport à l'arbre d'entraînement de la pompe de perfusion soit possible.

9. Seringue à piston selon la revendication 8, **caractérisée en ce que** l'axe central du corps (1) forme une tangente à la zone d'enroulement du tambour récepteur (6).

10. Seringue à piston selon la revendication 8 ou 9, **caractérisée en ce que** la partie filetée du tambour récepteur (6) s'étend jusque dans la zone d'enroulement.

11. Seringue à piston selon l'une des revendications 8 à 10, **caractérisée en ce que** le guidage de broche (7) est formé d'un seul tenant avec la paroi d'extrémité frontale du corps (1) qui comporte l'ouverture de sortie (11), et fait saillie depuis celle-ci.

12. Seringue à piston selon l'une des revendications 8 à 11, **caractérisée en ce que** l'orifice de distribution (11) du contenu de la seringue est prévu de façon excentrée dans la paroi d'extrémité frontale du corps (1).

13. Seringue à piston selon l'une des revendications 8 à 12, **caractérisée en ce que** l'élément de traction est un fil (5).

14. Seringue à piston selon l'une des revendications 8 à 13, **caractérisée en ce que** le piston (2, 3) est composé d'une partie avant (2) venant en contact étanche avec la paroi du corps, faite en une matière présentant des propriétés similaires à celles du caoutchouc, et d'une partie arrière rigide (3) qui est solidement reliée à la première.

15. Seringue à piston selon la revendication 14, **caractérisée en ce que** la partie arrière (3) du piston (2, 3) peut être reliée par conjugaison des formes à une tige de piston (4) permettant de tirer le piston en arrière.

16. Seringue à piston selon la revendication 15, **caractérisée en ce que**, dans une position de départ à l'état vide, le piston se trouve en contiguïté avec la paroi d'extrémité frontale du corps (1), et que la partie de l'élément de traction (5) qui, lorsque le piston est entièrement reculé, s'étend à l'intérieur du corps (1), se trouve entre le piston et la paroi d'extrémité frontale du corps (1), lorsque l'ensemble est dans la position de départ à l'état vide.
